Europäisches Patentamt

European Patent Office

Office européen des brevets

(11) Veröffentlichungsnummer: **0 230 021 B1**

(12) **EUROPÄISCHE PATENTSCHRIFT**

(45) Veröffentlichungstag der Patentschrift: 03.07.91

(51) Int. Cl.5: **C12P 7/40**, C12P 7/56, C12M 1/12

(21) Anmeldenummer: 86117736.8

(22) Anmeldetag: 19.12.86

(54) **Kontinuierliches Verfahren zur fermentativen Herstellung organischer Säuren.**

(30) Priorität: 20.12.85 JP 287521/85

(43) Veröffentlichungstag der Anmeldung:
29.07.87 Patentblatt 87/31

(45) Bekanntmachung des Hinweises auf die
Patenterteilung:
03.07.91 Patentblatt 91/27

(84) Benannte Vertragsstaaten:
AT BE CH DE ES FR GB IT LI NL SE

(56) Entgegenhaltungen:
DE-A- 1 957 395
FR-A- 1 564 115
US-A- 110 175
US-A- 2 829 095

CHEMICAL ABSTRACTS, Band 84, Nr. 21, 24.
Mai 1976, Seite 437, Zusammenfassung Nr.
149221q, Columbus, Ohio, US

(73) Patentinhaber: **HÜLS AKTIENGESELLSCHAFT**
**Paul-Baumann-Strasse 1**
**W-4370 Marl 1(DE)**

(72) Erfinder: **Czytko, Michael, Dr.**
**Hustardtring 25**
**W-4630 Bochum(DE)**
Erfinder: **Ishii, Kiyoshi**
**500 Kamivobe, Yobe-ku**
**Himeji 671-12(JP)**
Erfinder: **Kawai, Kimitoshi**
**906 Katajima, Ibogawa-cho**
**Ibo-gun, Hyogo, 671-16(JP)**

(74) Vertreter: **Patentanwälte Grünecker, Kinkel-**
**dey, Stockmair & Partner**
**Maximilianstrasse 58**
**W-8000 München 22(DE)**

APPLIED AND ENVIRONMENTAL MICROBIO-
LOGY, Band 52, Nr. 2, August 1986, Seiten
314-319, American Society for Microbiology;
M. HONGO et al.: "Novel method of lactic
acid production by electrodialysis fermentation"

PATENT ABSTRACTS OF JAPAN, Band 2, Nr.
38, November 1978, Seite 4538 C 77; & JP-A-
52 136 985 (AJINOMOTO K.K.) 16-11-1977

JOURNAL OF MEMBRANE SCIENCE, Band 27,
Nr. 2, Juni 1986, Seiten 165-171, Elsevier
Science Publishers B.V., New York, US; H.
VOSS: "Deacidification of citric acid solutions by electrodialysis"

CA88(11):73014j

## Beschreibung

Organische Säuren wie Milch-, Glucon-, Koji-, Citronen-, Äpfel-, Fumar- oder Itaconsäure können durch Fermentation aus Kohlenhydraten wie Stärke, Saccharose oder Glucose oder aus n-Paraffinen hergestellt werden. Dabei ist der Herstellungsprozeß der Säuren relativ einfach, während Isolierung und Reinigung der Produkte kompliziert und wenig effektiv sind.

So wird beispielsweise während der fermentativen Herstellung von Milchsäure $CaCO_3$ oder Ca-$(OH)_2$ zugesetzt, um den pH-Wert nicht absinken zu lassen. Nach der Reaktion werden die Zellen der Mikroorganismen filtriert, worauf durch Erhitzen gelöstes Protein koaguliert und ebenfalls filtriert wird. Daraufhin wird durch Zusatz von Schwefelsäure Milchsäure freigesetzt. Calciumsulfat wird gefällt und filtriert. Das Filtrat kann nun mit Aktivkohle oder mit Ionenaustauscher gereinigt werden. Aus der so erhaltenen Lösung gewinnt man Milchsäure durch Eindampfen. Teilweise wird Milchsäure noch in den Methylester übergeführt, worauf nach Destillation und Hydrolyse reine, freie Milchsäure gewonnen wird.

Zur Vereinfachung des Verfahrens zur Gewinnung von fermentativ hergestellten organischen Säuren wurde bereits die Verwendung von Ionentauschermembranen vorgeschlagen.

So beschreibt die japanische Patentschrift Nr. 50 958/1981 eine kontinuierliche Fermentation zur Herstellung von Itaconsäure, wobei in einem konstanten Strom Fermentationsbrühe abgenommen wird, aus dem dann die Zellen abfiltriert und hochmolekulare Verunreinigungen durch Ultrafiltration entfernt werden. In der Lösung wird dann Itaconsäure in ein Alkalisalz überführt. Daraufhin wird Itaconat durch Elektrodialyse abgetrennt und die Lösung in den Fermenter zurückgeführt. Man erhält so eine Itaconat-Lösung, aus der durch Ansäuern reine Itaconsäure gefällt werden kann. Es wird erwähnt, daß durch die kontinuierliche Entnahme von Produkt eine Zunahme von Verunreinigungen, die die Fermentation beeinträchtigen könnten, vermieden wird.

In der japanischen Patentschrift Nr. 32 959/1983 werden die Feststoffe bei der fermentativen Herstellung von Glutaminsäure filtriert, worauf im Filtrat Glutaminsäure in ein Alkalisalz übergeführt und anschließend durch Elektrodialyse abgetrennt wird.

Bei allen Verfahren werden jedoch die organischen Säuren in Form ihrer Salze gewonnen. Deshalb ist eine pH-Regulierung der Fermentationsbrühe durch Zugabe von Alkali notwendig. Wenn das gewünschte Endprodukt ein Salz einer organischen Säure ist, ist dieses Verfahren befriedigend. Ist jedoch das gewünschte Endprodukt eine freie Säure, wird als weiterer Reaktionsschritt eine Säurefreisetzung benötigt. Nachteilig daran ist, daß sich dadurch die Anzahl der Reaktionsschritte erhöht.

Aufgabe der vorliegenden Erfindung ist es, ein vereinfachtes Verfahren zur Fermentation von organischen Säuren bereitzustellen, bei dem

- aus einer Elektrodialyse-Einheit, die kontinuierlich mit Fermentationsbrühe versorgt wird, freie organische Säuren abgezogen werden,
- eine Produktinhibierung der Fermentation vermieden wird,
- eine pH-Einstellung durch Alkali während der Fermentation nicht erforderlich ist und
- eine Fermentation kontinuierlich und mit hoher Produktivität durchgeführt werden kann.

Erfindungsgemäß wird die Aufgabe durch ein kontinuierliches Fermentationsverfahren zur Herstellung organischer Säuren gelöst, bei dem ein Teil der Fermentationsbrühe vom Fermenter durch eine Elektrodialyse-Einheit und zum Fermenter zurück zirkuliert und bei dem die organischen Säuren als konzentrierte wäßrige Lösungen erhalten werden.

Die organischen Säuren gewinnt man durch Fermentation von Kohlenhydraten wie Stärke, Saccharose oder Glucose oder von n-Paraffinen. Beispiele für derartige Säuren sind Milch-, Glucon-, Koji-, Citronen- und Itaconsäure.

Als Mikroorganismen, die die organischen Säuren produzieren, können unter anderem Streptococcus lactis, Lactobacillus delbrueckii, Gluconobacter roseus, Aspergillus niger, Citromyces pfefferianus, Brevibacterium flavum, Aspergillus flavus und Aspergillus itaconicus eingesetzt werden.

Die Fermentation kann in üblichen Nährmedien für Mikroorganismen, die Säuren herstellen, durchgeführt werden. Diese Medien enthalten notwendigerweise mindestens ein Kohlenhydrat wie Glucose, Fructose, Saccharose und Maltose oder andere Stärkehydrolysate oder Substanzen, die Kohlenhydrate enthalten, wie beispielsweise Melasse. Sie enthalten außerdem anorganische Salze wie Magnesiumsulfat, $KH_2PO_4$ und gegebenenfalls Eisensulfat und mindestens eine wachstumsfördernde Komponente wie Hefeextrakt, Peptone, Fleischextrakt oder Sojabohnenpulver.

Bei der Fermentation wählt man eine für den Mikroorganismus geeignete Temperatur. Sie liegt im allgemeinen bei 25 bis 60 °C.

Die Mikroorganismen, die organische Säuren produzieren, sind säureempfindlich. Deshalb muß im Medium der pH-Wert zwischen 3 und 9 liegen.

In der Elektrodialyse-Einheit können handelsübliche Kationen- und Anionentauschermembranen verwendet werden. Beispiele für Kationentauschermembranen sind NEOSEPTA[R] CL-25 T, NEOSEPTA[R] CMS (Tokuyama Soda Co., Japan) und SELEMION CMV (Asahi Glass Co., Japan). Beispiele für Anionentauschermembranen sind NEOSEPTA[R] ACH-45 T, NEOSEPTA[R] AM-3, SELEMION[R] AMV, SELEMION[R] AMP und SELEMION[R] AMF.

NEOSEPTA[R] CMS und NEOSEPTA[R] AM-3 werden bevorzugt, da sie neben den organischen Säuren andere neutrale Moleküle sehr langsam hindurchdiffundieren lassen. Als Abschluß der Kathodenkammern werden SELEMION[R] AMP und SELEMION[R] AMF bevorzugt, da sie besonders alkalibeständig sind.

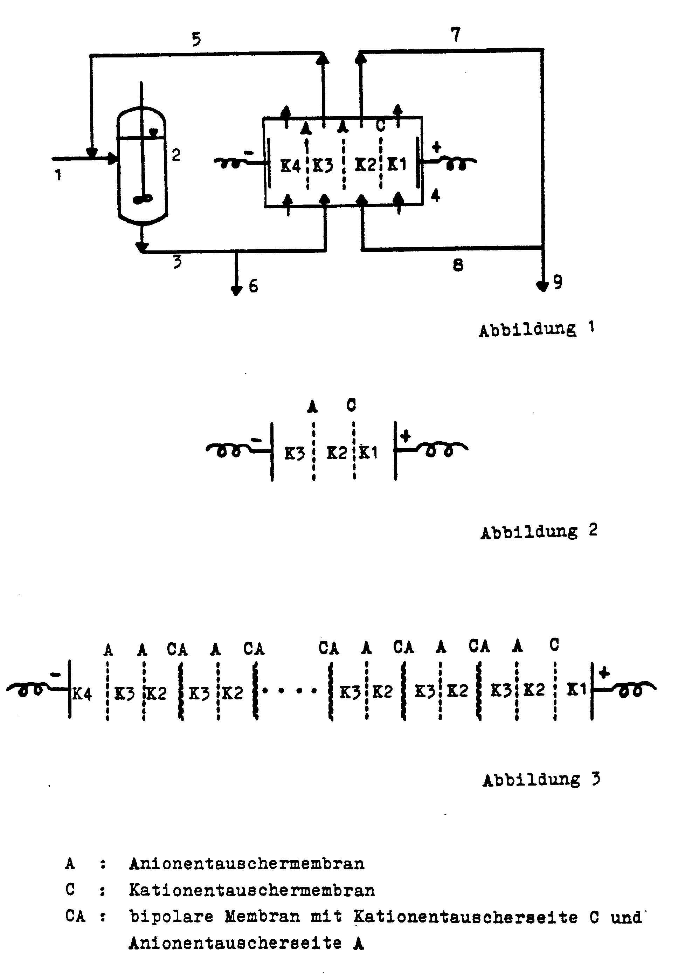

Die für die Elektrodialyse verwendeten Elektrodialyse-Einheiten sind in den Abbildungen 1 bis 3 schematisch dargestellt.

In Abbildung 1 wird eine Elektrodialyse-Einheit (4) mit 4 Kammern gezeigt, die, von der Anode aus gesehen, durch eine Kationentauschermembran (C) und 2 Anionentauschermembranen (A) voneinander getrennt sind. Eine wäßrige Säure wird durch die Anodenkammer K1 und eine wäßrige alkalische Lösung durch die Kathodenkammer K4 geleitet. Durch Kammer K3 zirkuliert Fermentationsbrühe und durch Kammer K2 eine Lösung der Produktsäure.

Der Fermenter (2) kann mit einer pH-Elektrode ausgerüstet sein, durch die die Stromstärke in der Elektrodialyse-Einheit (4) gesteuert wird. So kann die Stromstärke automatisch erhöht werden, wenn die Konzentration der gebildeten Säure ansteigt und ein für die Fermentation geeigneter pH-Wert unterschritten wird. Auf der anderen Seite kann auch der Strom automatisch abgestellt werden, wenn ein bestimmter hoher pH-Wert in der Fermentationsbrühe erreicht wird.

Nach Anlegen eines elektrischen Feldes an die Elektrodialyse-Einheit wandern Anionen der gebildeten Säure von K3 nach K2, wo sie mit Protonen, die von K1 nach K2 gewandert sind, neutralisiert werden. Auf diese Weise erhält man in K2 die im Fermenter gebildete organische Säure als wäßrige freie Säure. Über Leitung (9) wird ein Teil der Lösung kontinuierlich abgenommen, worauf die organische Säure nach bekannten Methoden gewonnen wird.

In K3 werden die Säureanionen, die nach K2 gewandert sind, durch Hydroxylionen, die von K4 nach K3 gewandert sind, ersetzt. Die Fermentationsbrühe wird, nach Zudosierung von Substrat und Nährlösung über Leitung (1), in den Fermenter zurückgeführt.

Zur Regulierung der Füllstandshöhe im Fermenter und der Zelldichte in der Fermentationsbrühe wird ein Teil der Brühe über Leitung (6) ausgeschleust. Dabei können die Zellen des Mikroorganismus ggf. durch Kreuzstrom-Mikrofiltration zurückgehalten und zurückgeführt werden, wobei dann lediglich zellfreies Permeat abgetrennt wird.

Abbildung 2 zeigt eine Elektrodialyse-Einheit mit nur 3 Kammern, wobei durch K1, K2 und K3 die gleichen Lösungen zirkulieren wie in Abbildung 1. In Abbildung 2 entfällt Kammer K4 für eine alkalische Lösung.

K4, die Kathodenkammer von Abbildung 1, dient dazu, Hydroxylionen zu produzieren, die in K3 die abgewanderten Säureanionen ersetzen sollen. K4 wird benötigt, wenn der Kontakt der Kathode mit der Fermentationsbrühe Schwierigkeiten verursacht. Gibt es dagegen keine Probleme bei einem direkten Kontakt der Fermentationsbrühe mit der Kathode, so kann die Brühe auch durch die Kathodenkammer geleitet werden. Dann werden die Hydroxylionen direkt in der Brühe gebildet. Die Anionentauschermembran zwischen K3 und K4 und die alkalische Lösung können dadurch eingespart werden.

Abbildung 3 zeigt schematisch eine Serie von Elektrodialyse-Einheiten, wobei durch K1 und K4 sowie durch die Serie von Paaren aus K2 und K3 die gleichen Lösungen zirkulieren wie in Abbildung 1.

Nach Anlegen eines elektrischen Feldes werden aus den bipolaren Membranen (CA) in die Kammern K2 Protonen und in die Kammern K3 Hydroxylionen geliefert.

Wenn die Bakterien zur Belagbildung auf den Membranen neigen, können die Bakterien aus der Fermentationsbrühe durch eine Kreuzstrom-Mikrofiltration herausgefiltert werden, so daß dann nur eine mikroorganismenfreie Fermentationsbrühe durch die Elektrodialyse-Einheit geleitet wird. Auf diese Art wird der Kreislauf in 2 Kreisläufe aufgespalten, einen bakterienhaltigen durch Fermenter und Mikrofiltrationsstufe und einen bakterienfreien durch Mikrofiltrationsstufe und Elektrodialyse-Einheit.

Das erfindungsgemäße Verfahren erfordert keinen Zusatz von Alkali. Eine Erniedrigung des pH-Wertes in der Fermentationsbrühe wird nur durch die Abtrennung von Produktsäure in der Elektrodialyse-Einheit ausgeglichen.

Die Fermentationsbrühe wird im Kreise geführt und lediglich durch Zudosierung von Substrat und Nährlösung ergänzt. Auf der anderen Seite wird nur Produktsäurelösung abgetrennt. So entsteht ein geschlossener Kreislauf, in dem eine kontinuierliche Fermentation durchgeführt wird.

Da die Fermentationsbrühe mit den Mikroorganismen durch die Elektrodialyse-Einheit geleitet wird, werden möglicherweise vorhandene fremde Mikroorganismen am Wachstum gehindert, so daß sie keinen Einfluß auf die Fermentation gewinnen. Obwohl zu Beginn der Fermentation Fermenter und Fermentationsmedium sterilisiert werden müssen, um eine Kontamination zu vermeiden, ist bei gut laufender Fermentation eine Sterilisation der Nährlösung nicht mehr erforderlich.

Die Vorteile der Erfindung:

1) Eine kontinuierliche Fermentation kann mit einer hohen Produktivität an organischer Säure von 30 g/l·h oder mehr durchgeführt werden.

2) Eine wäßrige Lösung einer freien organischen Säure kann mit hoher Konzentration und hoher Reinheit einfach und effektiv aus der organischen Säure der Fermentationsbrühe gewonnen werden.

3) Eine Konzentration der organischen Säure von 30 % oder mehr kann erhalten werden.

4) Eine hohe Stromausbeute von 90 % oder mehr kann bei der Elektrodialyse erreicht werden.

5) Arbeit und Kosten für die Entfernung der Mikroorganismen aus der Fermentationsbrühe können gespart werden.

6) Wachstum von fremden, kontaminierenden Bakterien kann durch die Zirkulation der Fermentationsbrühe durch die Elektrodialyseeinheit unterdrückt werden.

7) Eine Verwendung von Neutralisationsagentien (Basen) zur Einhaltung eines geeigneten pH-Wertes der Fermentationsbrühe ist nicht nötig.

Die Beispiele sollen die Erfindung verdeutlichen.

Beispiel 1

In einem 0,7 l-Glasfermenter mit Rührer, Temperaturkontrolle und pH-Wert-Messung werden zur Anzucht 360 ml Nährlösung I mit

$$35 \ \text{g/l Glucose}$$
$$10 \ \text{g/l Hefeextrakt}$$
$$0,6 \ \text{g/l MgSO}_4 \cdot 7 \ \text{H}_2\text{O}$$
$$0,03 \ \text{g/l MnSO}_4 \cdot 5 \ \text{H}_2\text{O}$$
$$0,03 \ \text{g/l FeSO}_4 \cdot 4 \ \text{H}_2\text{O}$$
$$1 \ \text{g/l K}_2\text{HPO}_4$$
$$1 \ \text{g/l KH}_2\text{PO}_4$$

gegeben. Die Lösung beimpft man mit 50 ml Inoculum, das wie Nährlösung I zusammengesetzt ist und außerdem etwa 0,1 g Zellmasse von Lactobacillus delbrueckii NRRL-B 445 enthält. Nun rührt man bei 42 °C unter anaeroben Bedingungen.

Nach 9 h beträgt die Zelldichte des Mikroorganismus (Trockengewicht) 3,2 g/l. Es sind 25 g/l Milchsäure gebildet worden, und es liegen noch 7 g/l Glucose vor.

Nun gibt man 0,1 bis 0,6 ml/min Nährlösung II, die

$$378 \ \text{g/l Glucose}$$
$$61 \ \text{g/l Hefeextrakt}$$
$$1,5 \ \text{g/l MgSO}_4 \cdot 7 \ \text{H}_2\text{O}$$
$$0,1 \ \text{g/l MnSO}_4 \cdot 5 \ \text{H}_2\text{O}$$
$$0,1 \ \text{g/l FeSO}_4 \cdot 4 \ \text{H}_2\text{O}$$
$$1 \ \text{g/l K}_2\text{HPO}_4$$
$$1 \ \text{g/l KH}_2\text{PO}_4$$

enthält, in den Fermenter und läßt die Fermentationsbrühe mit 17 l/h durch ein Elektrodialysegerät gemäß Abbildung 1 und zurück zum Fermenter zirkulieren.

Daten des Elektrodialysegerätes:
1 Kationentauschermembran NEOSEPTA[R] CMS,
2 Anionentauschermembranen NEOSEPTA[R] AM-3, wirksame Membranfläche jeweils 1,7 dm².

Durch die 4 Kammern der Elektrodialyseeinheit zirkulieren folgende wäßrige Lösungen:
Durch
K1: 1,8 %ige $H_2SO_4$
K2: 3 %ige Milchsäure (Anfangswert)
K3: Fermentationsbrühe vom Fermenter
K4: 0,2 %ige NaOH

Die Elektrodialyse wird über die pH-Wert-Messung so geregelt, daß im Fermenter ein pH-Wert von 6 ± 0,1 gehalten wird. Die Stromstärke beträgt am Anfang der kontinuierlichen Fermentation 1,3 Ampere, beim Übergang in die stationäre Phase 5,1 Ampere.

Immer wenn der Strom für die Elektrodialyseeinheit eingeschaltet ist, nimmt das Volumen der Milchsäurelösung im Kreis durch Kammer K2 zu. Der Überschuß an Milchsäurelösung wird aus diesem Kreis über ein Überlaufrohr abgetrennt. Aus dieser überschüssigen Lösung wird reine Milchsäure durch Destillation gewonnen.

Während der kontinuierlichen Fermentation wird etwas Fermentationsbrühe (etwa 0,05 bis 0,4 ml/min) aus dem Kreis abgetrennt. Nach 6 Stunden liegt die Produktivität bei 28 g Milchsäure pro Liter Fermentationsbrühe und Stunde und die Zelldichte bei 8,2 g/l (Trockenmasse). Im Produktsäurekreis liegt die Konzentration bei 173 g Milchsäure pro Liter Lösung.
Milchsäureausbeute, bezogen auf Glucose: 95 %
Stromausbeute: 80%

Beispiel 2

Die Anzucht des Mikroorganismus Lactobacillus delbrueckii NRRL-B 445 erfolgt wie in Beispiel 1.

Nach Beginn der Zudosierung von 0,1 bis 0,7 ml/min Nährlösung II läßt man die Fermentationsbrühe mit 17 l/h durch ein Elektrodialysegerät gemäß Abbildung 1 zirkulieren.

Daten des Elektrodialysegerätes:
1 Kationentauschermembran NEOSEPTA$^R$ CMS,
1 Anionentauschermembran NEOSEPTA$^R$ AM-3 (K2/K3),
1 Anionentauschermembran SELEMION$^R$ AMP (K3/K4),
wirksame Membranfläche jeweils 1,7 dm$^2$.

Durch die 4 Kammern der Elektrodialyse-Einheit zirkulieren folgende wäßrige Lösungen:
Durch
K1: 1,8 %ige H$_2$SO$_4$
K2: 5 %ige Milchsäure (Anfangswert)
K3: Fermentationsbrühe vom Fermenter
K4: 0,9 %ige NaOH

Am Anfang der kontinuierlichen Fermentation beträgt die Stromstärke 1,4 Ampere. Nach 14 Stunden wird eine stationäre Phase erreicht, und die Stromstärke beträgt 5,4 Ampere. Nach 14 Stunden ist die Konzentration der Zellmasse in der Fermentationsbrühe auf 11 g/l angestiegen (berechnet als Trockenmasse).

Überschüssige Milchsäure wird in der gleichen Weise wie in Beispiel 1 abgetrennt.

Während der kontinuierlichen Fermentation wird etwas Fermentationsbrühe (etwa 0,05 bis 0,4 ml/min) aus dem Kreis abgetrennt.

Unter stationären Bedingungen beträgt die Produktivität 35 g Milchsäure pro Liter Fermentationsbrühe und Stunde. Im Produktsäurekreis liegt die Konzentration bei 240 g Milchsäure pro Liter Lösung.
Milchsäureausbeute, bezogen auf Glucose: 96 %
Stromausbeute: 93%

Die Glucosekonzentration in der Fermentationsbrühe wird bei 2 g/l oder mehr gehalten. Eine Sterilisation der zugeführten Nährlösung II wird nicht durchgeführt. Wachstum von fremden Bakterien wird nicht beobachtet.

Dieses beweist, daß der bakterizide Effekt der Elektrodialyse stark genug ist, um durch Kontamination eingeführte fremde Bakterien zu unterdrükken. Gleichzeitig ist dieser Effekt nicht so groß, um die normale Aktivität der Hauptbakterien (Lactobacillus delbrueckii) zu beeinflussen. Es werden deshalb keine problematischen Antikontaminationsvorrichtungen benötigt.

Beispiel 3

Die Anzucht von Lactobacillus delbrueckii NRRL-B 445 erfolgt wie in Beispiel 1.

Nach Beginn der Zudosierung von 0,1 bis 0,6

ml/min Nährlösung II wird Fermentationslösung mit 17 l/h durch ein Elektrodialysegerät gemäß Abbildung 2 geleitet.

Daten des Elektrodialysegerätes:
1 Kationentauschermembran NEOSEPTA$^R$ CMS,
1 Anionentauschermembran NEOSEPTA$^R$ AM-3,
wirksame Membranfläche jeweils 1,7 dm$^2$.

Durch die 3 Kammern der Elektrodialyseeinheit zirkulieren folgende wäßrige Lösungen:
Durch
K1: 1,8 %ige H$_2$SO$_4$
K2: 5 %ige Milchsäure (Anfangswert)
K3: Fermentationsbrühe vom Fermenter

Die Stromversorgung für das Elektrodialysegerät wird an- und abgeschaltet wie in Beispiel 1.

Nach 13 Stunden wird eine Zelldichte (Trockengewicht) von 16 g/l erreicht.

Überschüssige Milchsäurelösung und etwas Fermentationsbrühe werden in der gleichen Weise wie in Beispiel 1 abgetrennt.

Unter stationären Bedingungen beträgt die Produktivität 19 g Milchsäure pro Liter Fermentationsbrühe und Stunde. Im Produktsäurekreis liegt die Konzentration bei 187 g Milchsäure pro Liter Lösung.
Milchsäureausbeute, bezogen auf Glucose: 84 %
Stromausbeute: 70 %

Die Glucosekonzentration in der Fermentationsbrühe wird bei 2 g/l oder mehr gehalten.

**Ansprüche**

1. Kontinuierliches Verfahren zur fermentativen Herstellung organischer Säuren, dadurch gekennzeichnet, daß man die Fermentationsbrühe vom Fermenter durch eine Elektrodialyse-Einheit und zurück zum Fermenter führt und dabei eine Lösung der organischen Säuren gewinnt.

2. Verfahren nach Anspruch 1, dadurch gekennzeichnet, daß man die Elektrodialyse in einer Elektrodialyse-Einheit mit 3 Kammern durchführt, die, von der Anode aus gesehen, durch eine Kationentauscher- und eine Anionentauschermembran voneinander getrennt sind.

3. Verfahren nach Anspruch 2, dadurch gekennzeichnet, daß man gemäß Abbildung 2 eine Säure durch Kammer K1, die Produktsäure durch Kammer K2 und die Fermentationsbrühe durch Kammer K3 zirkulieren läßt und aus der Lösung, die durch Kammer K2 fließt, die Produktsäure gewinnt.

4. Verfahren nach Anspruch 1,

dadurch gekennzeichnet,
daß man die Elektrodialyse in einer Elektrodialyse-Einheit mit 4 Kammern durchführt, die, von der Anode aus gesehen, durch eine Kationentauschermembran und 2 Anionentauschermembranen voneinander getrennt sind.

5. Verfahren nach Anspruch 4,
dadurch gekennzeichnet,
daß man gemäß Abbildung 1 eine Säure durch Kammer K1, die Produktsäure durch Kammer K2, die Fermentationsbrühe durch Kammer K3 und eine alkalische Lösung durch Kammer K4 zirkulieren läßt und aus der Lösung, die durch Kammer K2 fließt, die Säure gewinnt.

6. Verfahren nach Anspruch 1,
dadurch gekennzeichnet,
daß man die Elektrodialyse in einer Serie von Elektrodialyse-Einheiten durchführt, die eine Anodenkammer K1, eine Serie von Paaren aus Kammern K2 und K3 und eine Kathodenkammer K4 aufweisen, die, von der Anode aus gesehen, durch eine Kationentauschermembran, eine Anionentauschermembran, eine Serie von Paaren aus bipolarer Membran und Anionentauschermembran und durch eine Anionentauschermembran voneinander getrennt sind.

7. Verfahren nach Anspruch 6,
dadurch gekennzeichnet,
daß man gemäß Abbildung 3 eine Säure durch Kammer K1, die Produktsäure durch die Kammern K2, die Fermentationsbrühe durch die Kammern K3 und eine alkalische Lösung durch Kammer K4 zirkulieren läßt und aus der Lösung, die durch die Kammern K2 fließt, die Säure abtrennt.

8. Verfahren nach den Ansprüchen 6 und 7,
dadurch gekennzeichnet,
daß man die Elektrodialyse in einem Gerät mit 10 bis 100 Paaren von Kammern K2 und K3 durchführt.

9. Verfahren nach den Ansprüchen 3, 5 und 7,
dadurch gekennzeichnet,
daß die Säure in Kammer K1 eine 0,1 bis 10 %ige Schwefelsäure und die alkalische Lösung in Kammer K4 eine 0,01 bis 10 %ige Natronlauge ist.

10. Verfahren nach den Ansprüchen 1 bis 9 ,
dadurch gekenzeichnet,
daß als Produktsäure Milchsäure gewonnen wird.

11. Verfahren nach Anspruch 1,
dadurch gekennzeichnet,
daß man die Fermentationsbrühe einer Mikrofiltration unterwirft und eine mikroorganismenfreie Fermentationsbrühe durch eine Elektrodialyse-Einheit und zurück zum Fermenter leitet.

**Claims**

1. Continuous process for the production of organic acids by means of fermentation, characterised in that the fermentation stock is removed from, and returned to the fermenter by means of an electro-dialysis unit, by which method a solution of the organic acids is produced.

2. Process according to Claim 1, characterised in that the electro-dialysis is performed in an electro-dialysis unit comprising 3 chambers which, as seen from the anode, are separated from each other by a cation exchanger - and an anion exchanger diaphragm.

3. Process according to Claim 2, characterised in that an acid is circulated through chamber K1, the product acid through chamber K2 and the fermentation stock through chamber K3, and that the product acid is won from the solution which flows through chamber K2, as shown in illustration 2.

4. Process according to Claim 1, characterised in that the electro-dialysis is performed in an electro-dialysis unit comprising 4 chambers which, as seen from the anode, are separated from each other by a cation exchanger and 2 anion exchanger diaphragms.

5. Process according to Claim 4, characterised in that an acid is circulated through chamber K1, the product acid through chamber K2, the fermentation stock through chamber K3, and an alkaline solution through chamber K4, and that the acid is won from the solution which flows through chamber K2, as shown in illustration 1.

6. Process according to Claim 1, characterised in that the electro-dialysis is performed in a series of electro-dialysis units, comprising an anode chamber K1, a series of pairs of chambers K2 and K3 and a cathode chamber K4, which, as seen from the anode, are separated from each other by a cation exchanger diaphragm, an anion exchanger diaphragm, a series of pairs of bipolar diaphragms and anion exchanger diaphragms, and by an anion exchanger diaphragm.

7. Process according to Claim 6, characterised in that an acid is circulated through a chamber K1, the product acid through chambers K2, the fermentation stock through chambers K3, and an alkaline solution through chamber K4, and in that the acid is separated from the solution which flows through chambers K2, as shown in illustration 3.

8. Process according to Claims 6 and 7, characterised in that the electro-dialysis is performed in an apparatus comprising 10 to 100 pairs of chambers K2 and K3.

9. Process according to Claims 3, 5 and 7, characterised in that the acid in chamber K1 is a 0.1 to 10% sulphuric acid, and the alkaline solution in chamber K4 is a 0.01 to 10% caustic soda solution.

10. Process according to Claims 1 to 9, characterised in that the obtained product acid is lactic acid.

11. Process according to Claim 1, characterised in that the fermentation stock is subjected to micro filtration, and in that a fermentation stock, which is free of micro-organisms, is conveyed through an electro-dialysis unit and back to the fermenter.

**Revendications**

1. Procédé continu pour la préparation par fermentation d'acides organiques, caractérisé en ce qu'on envoie le bouillon de fermentation depuis le fermenteur à travers un dispositif d'électrodialyse puis on le renvoie au fermenteur et on obtient ainsi une solution des acides organiques.

2. Procédé selon la revendication 1, caractérisé en ce qu'on effectue l'électrodialyse dans un dispositif d'électrodialyse à trois chambres, qui, vues à partir de l'anode, sont séparées les unes des autres par une membrane échangeuse de cations et par une membrane échangeuse d'anions.

3. Procédé selon la revendication 2, caractérisé en ce que, conformément à la figure 2, on fait circuler un acide à travers la chambre K1, l'acide produit à travers la chambre K2 et le bouillon de fermentation à travers la chambre K3 et en ce qu'on obtient l'acide produit à partir de la solution qui s'écoule à travers la chambre K2.

4. Procédé selon la revendication 1, caractérisé en ce qu'on effectue l'électrodialyse dans un dispositif d'électrodialyse à quatre chambres, qui, vues de l'anode, sont séparées les unes des autres par une membrane échangeuse de cations et par deux membranes échangeuses d'anions.

5. Procédé selon la revendication 4, caractérisé en ce que, conformément à la figure 1, on fait circuler un acide à travers la chambre K1, l'acide produit à travers la chambre K2, le bouillon de fermentation à travers la chambre K3 et une solution alcaline à travers la chambre K4 et en ce qu'on obtient l'acide à partir de la solution qui s'écoule à travers la chambre K2.

6. Procédé selon la revendication 1, caractérisé en ce qu'on effectue l'électrodialyse dans une série de dispositifs d'électrodialyse qui présentent une chambre anodique K1, une série de paires de chambres K2 et K3 et une chambre cathodique K4, qui, vues de l'anode, sont séparées les unes des autres par une membrane échangeuse de cations, une membrane échangeuse d'anions, une série de paires de membranes bipolaires et de membrane échangeuse d'anions, et par une membrane échangeuse d'anions.

7. Procédé selon la revendication 6, caractérisé en ce que conformément à la figure 3, on fait circuler un acide à travers la chambre K1, l'acide produit à travers les chambres K2, le bouillon de fermentation à travers les chambres K3 et une solution alcaline à travers la chambre K4 et en ce qu'on sépare l'acide de la solution qui s'écoule à travers les chambres K2.

8. Procédé selon les revendications 6 et 7, caractérisé en ce qu'on effectue l'électrodialyse dans un appareil comportant 10 à 100 paires de chambres K2 et K3.

9. Procédé selon les revendications 3, 5 et 7. caractérisé en ce que l'acide présent dans la chambre K1 est un acide sulfurique à 0,1 à 10% et en ce que la solution alcaline présente dans la chambre K4 est une lessive de soude de 0,01 à 10%.

10. Procédé selon les revendications 1 à 9, caractérisé en ce que l'on obtient, comme acide produit, de l'acide lactique.

**11.** Procédé selon la revendication 1, caractérisé en ce qu'on soumet le bouillon de fermentation à une microfiltration et en ce qu'on envoie un bouillon de fermentation exempt de micro-organisme à travers un dispositif d'électrodialyse et en ce qu'on le renvoie au fermenteur.

Abbildung 1

Abbildung 2

Abbildung 3

A : Anionentauschermembran

C : Kationentauschermembran

CA : bipolare Membran mit Kationentauscherseite C und
Anionentauscherseite A